# EUROPEAN PATENT SPECIFICATION

(11) **EP 3 604 274 B1**
(45) Date of publication and mention of the grant of the patent: **11.11.2026**
(21) Application number: 18778012.7
(22) Date of filing: 30.03.2018
(51) Int. Cl.: C11D 1/04, C11D 1/12, B01F 23/41, B01F 23/50

(54) **USES OF SALTS OF SULFONIC ACID AND CARBOXYLIC ACID DERIVATIVES**
VERWENDUNG VON SALZEN VON SULFONSÄUREDERIVATEN UND CARBONSÄUREDERIVATEN
UTILISATIONS DE SELS DE DÉRIVÉS D'ACIDE SULFONIQUE ET DE DÉRIVÉS D'ACIDE CARBOXYLIQUE

(30) Priority: 31.03.2017 JP 2017073086
(43) Date of publication of application: 05.02.2020
(73) Proprietor: Daikin Industries, Ltd., Osaka-shi, Osaka 530-0001 (JP)
(72) Inventor: HIGASHI, Masahiro, Osaka-Shi Osaka 530-8323 (JP); YONEDA, Satoru, Osaka-Shi Osaka 530-8323 (JP); YAMAUCHI, Akiyoshi, Osaka-Shi Osaka 530-8323 (JP); ISHIHARA, Sumi, Osaka-Shi Osaka 530-8323 (JP); KISHIKAWA, Yosuke, Osaka-Shi Osaka 530-8323 (JP); NAKANO, Marina, Osaka-Shi Osaka 530-8323 (JP); NITTA, Shinnosuke, Osaka-Shi Osaka 530-8323 (JP); AOYAMA, Hirokazu, Osaka-Shi Osaka 530-8323 (JP)
(74) Representative: Hoffmann Eitle
(86) International application number: PCT/JP2018/013635
(87) International publication number: WO 2018/181907

(56) References cited:
- WO-A1-2013/045662
- WO-A1-2013/045662
- WO-A1-2015/012107
- GB-A- 1 081 234
- JP-A- 2003 005 329
- JP-A- 2003 005 329
- JP-A- H09 503 295
- JP-A- S4 929 294
- JP-A- S4 929 294
- US-A- 5 043 340
- US-A1- 2016 037 034
- US-A1- 2016 155 969
- M. KITAMURA, ET AL.: "Asymmetric synthesis of beta-hydroxy sulfonic acids by BINAP/Ru-catalysed hydrogenation", TETRAHEDRON, vol. 55, no. 29, 16 July 1999 (1999-07-16), Elsevier Science Publishers, Oxford, GB, pages 8769 - 8785, XP004171385, ISSN: 0040-4020, DOI: 10.1016/S0040-4020(99)00443-3
- S. HIRANUMA, ET AL.: "Synthesis of homoharringtonine and its derivative by partial esterification of cephalotaxine", TETRAHEDRON LETTERS, vol. 23, no. 34, August 1982 (1982-08-01), Elsevier Science Publishers, Oxford, GB, pages 3431 - 3434, XP002163588, ISSN: 0040-4039, DOI: 10.1016/S0040-4039(00)87634-0
- M. KRASAVIN, ET AL.: "New nitrofurans amenable by isocyanide multicomponent chemistry are active against multidrug-resistant and poly-resistant Mycobacterium tuberculosis", BIOORGANIC & MEDICINAL CHEMISTRY, vol. 25, no. 6, 4 February 2017 (2017-02-04), Elsevier Science Publishers, Oxford, GB, pages 1867 - 1874, XP055754616, ISSN: 0968-0896, DOI: 10.1016/j.bmc.2017.02.003
- K. ISHIHARA, ET AL.: "Highly diastereoselective acetal cleavages using novel reagents prepared from organoaluminium and pentafluorophenol", JOURNAL OF THE AMERICAN CHEMICAL SOCIETY, vol. 115, 1 November 1993 (1993-11-01), American Chemical Society, Washington, DC, US, pages 10695 - 10704, XP000930002, ISSN: 0002-7863, DOI: 10.1021/ja00076a030
- S.M. OSMAN, ET AL.: "Markonikov hydration and amination of 10-undecenoic acid and BF3-catalysed dimethyl sulfoxide oxidation of its methyl epoxide", FETTE, SEIFEN, ANSTRICHMITTEL, vol. 77, no. 3, March 1975 (1975-03-01), Wiley-VCH Verlag, Weinheim, DE, pages 106 - 108, XP000644554, DOI: 10.1002/lipi.19750770306
- Y. NAOSHIMA, ET AL.: "Synthesis of both the enantiomers of phoracantholide I, a defensive secretion of the eucarypt longicorn (Phoracantha synonyma), employing microbial asymmetric reduction with immobilized baker's yeast", BULLETIN OF THE CHEMICAL SOCIETY OF JAPAN, vol. 62, no. 2, February 1989 (1989-02-01), Chemical Soceity of Japan, Tokyo, JP, pages 608 - 610, XP093104858, ISSN: 0009-2673, DOI: 10.1246/bcsj.62.608
- H.L. CASAL: "The water content of micelles: infrared spectroscopic studies", JOURNAL OF THE AMERICAN CHEMICAL SOCIETY, vol. 110, no. 15, 1 July 1988 (1988-07-01), American Chemical Society, Washington, DC, US, pages 5203 - 5208, XP093105128, ISSN: 0002-7863, DOI: 1021/ja00223a056

## Description

### TECHNICAL FIELD

The invention relates to the use of sulfonic acid and carboxylic acid derivatives as surfactants, surfactant accelerators, viscosity reducers, dispersants or emulsifiers.

### BACKGROUND ART

H.L. Casal, Journal of the American Chemical Society, 1988, 110(15), 5203-5205 reports of studies of the water content of micelles, using, inter alia, sodium 7-oxoactanoate.

WO 2013/045662 discloses a compound of the formula.

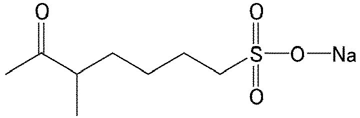

### SUMMARY OF INVENTION

### - Technical Problem

The invention aims to provide sulfonic acid and carboxylic acid derivatives useful as surfactants, surfactant accelerators, viscosity reducers, dispersants or emulsifiers.

### - Solution to Problem

The invention relates to the use of a compound of formula (I): 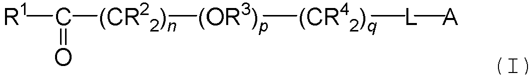 wherein
- R¹: is linear or branched C₁₋₄-alkyl;
- R², R⁴: each individually are H or linear or branched C₁₋₄-alkyl;
- R³: is unsubstituted C₁₋₄-alkylene;
- *n*: is an integer of ≥ 1;
- *p*, *q*: each individually are an integer of ≥ 0;
and(*n*+*p*+q) is an integer of ≥ 6;
- A: is -SO₃X or -COOX, wherein X is an alkali metal atom; an alkaline earth metal atom; NR⁵₄ wherein R⁵ each independently is H or an organic group; or imidazolium, pyridinium or phosphonium, each optionally substituted; and
- L: is a single bond, -CO₂-B-*, -OCO-B-*, -CONR⁶-B-*, - NR⁶CO-B-*, or -CO- other than the carbonyl groups in the aforementioned groups-, B is a single bond or optionally substituted C₁₋₁₀-alkylene, R⁶ is H or optionally substituted C₁₋₄-alkyl, and * indicates the bond to A in the formula;

and the compound contains ≤ 20 carbon atoms in total,
as a surfactant, surfactant accelerator, viscosity reducer, dispersant or emulsifier.

The compound preferably contains 3-20 carbon atoms in total.

L is preferably a single bond.

R¹ is preferably methyl.

X in the formula is preferably an alkali or alkaline earth metal atom or NR⁵₄, wherein R⁵ is defined as described above.

### - Advantageous Effects of Invention

The compound of formula (I) (also "the present compound" hereinafter) is a compound exhibiting a surfactant activity, and can suitably be used as an aqueous dispersant.

### DESCRIPTION OF EMBODIMENTS

The invention is described in detail below.

The term "organic group" as used herein means a group containing one or more carbon atoms or a group obtainable by removing one hydrogen atom from an organic compound, unless otherwise mentioned.

Examples of the "organic group" include alkyl, alkenyl, alkynyl, cycloalkyl, cycloalkenyl, cycloalkadienyl, aryl, aralkyl, a non-aromatic heterocyclic group and heteroaryl, each optionally containing one or more substituents;and
cyano, formyl, RaO-, RaCO-, RaSO₂-, RaCOO-, RaNRaCO-, RaCONRa-, RaOCO-, and RaOSO₂-,
wherein each Ra is independently alkyl, alkenyl, alkynyl, cycloalkyl, cycloalkenyl, cycloalkadienyl, aryl, aralkyl, a non-aromatic heterocyclic group or heteroaryl, each optionally containing one or more substituents.

The organic group is preferably alkyl optionally containing one or more substituents.

The term "substituent" as used herein means a group which can replace another atom or group, unless otherwise mentioned. Examples of the "substituent" include an aliphatic group, an aromatic group, a heterocyclic group, acyl, acyloxy, acylamino, aliphatic oxy, aromatic oxy, heterocyclic oxy, aliphatic oxycarbonyl, aromatic oxycarbonyl, heterocyclic oxycarbonyl, carbamoyl, aliphatic sulfonyl, aromatic sulfonyl, heterocyclic sulfonyl, aliphatic sulfonyloxy, aromatic sulfonyloxy, heterocyclic sulfonyloxy, sulfamoyl, aliphatic sulfonamide, aromatic sulfonamide, heterocyclic sulfonamide, amino, aliphatic amino, aromatic amino, heterocyclic amino, aliphatic oxycarbonylamino, aromatic oxycarbonylamino, heterocyclic oxycarbonylamino, aliphatic sulfinyl, aromatic sulfinyl, aliphatic thio, aromatic thio, hydroxy, cyano, sulfo, carboxy, aliphatic oxyamino, aromatic oxyamino, carbamoylamino, sulfamoyl amino, halogen, sulfamoyl carbamoyl, carbamoyl sulfamoyl, dialiphatic oxyphosphinyl, and diaromatic oxyphosphinyl.

The invention relates to the use of a compound of formula (I):

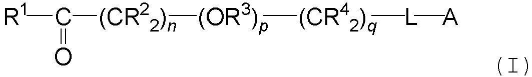

In the formula, R¹ is a linear or branched C₁₋₄-alkyl.

The compound preferably contains 3-20, more preferably 5-20, still more preferably 7-20 carbon atoms in total.

R¹ is preferably C1-C3 linear or branched C₁₋₃-alkyl free from a substituent, particularly preferably methyl (-CH₃) or ethyl (-C₂H₅), most preferably methyl .

In the formula, R² and R⁴ are each individually H or linear or branched C₁₋₄-alkyl; multiple R² may be the same as or different from each other and multiple R⁴ may be the same as or different from each other.

The alkyl group for each of R² and R⁴ is preferably linear or branched C₁₋₃-alkyl, particularly preferably methyl or ethyl .

R² and R⁴ are preferably each individually H or linear or branched C₁₋₃-alkyl free from a substituent, particularly preferably H, methyl or ethyl, most preferably H.

R³ is unsubstituted C₁₋₄-alkyleneWhen multiple R³ are present, they may be the same as or different from each other.

The alkylene group is preferably methylene (-CH₂-), ethylene (-C₂H₄-), isopropylene (-CH(CH₃)CH₂-) or propylene (-C₃H₆-).

In the formula, n is an integer of ≥ 1, preferably 1-40, more preferably 1-30, still more preferably 5-25.

In the formula, p and q are each individually an integer of ≥ 0; p is preferably an integer of 0-10, more preferably 0 or 1, while q is preferably an integer of 0-10, more preferably an integer of 0-5.

The sum of n, p, and q (n+p+q) is an integer of ≥ 6, preferably ≥ 8. (n+p+q) is also preferably an integer of ≤ 60, more preferably ≤ 50, still more preferably ≤ 40.

In the formula, A is -SO₃X or -COOX, wherein X is an alkali metal atom; an alkaline earth metal atom; NR⁵₄ wherein R⁵ each independently is H or an organic group; or imidazolium, pyridinium or phosphonium, each optionally containing a substituent. R⁵ is preferably H or a C₁₋₁₀-organic group, more preferably H or a C₁₋₄-organic group. Preferred examples of the alkali or alkaline earth metal are Na, K, or Li.

X is preferably Na, K, Li, or NH₄, further more preferably Na, K, or NH₄, particularly preferably Na or NH₄, most preferably NH₄.

In the formula, L is a single bond, -CO₂-B-*, -OCO-B-*, -CONR⁶-B-*, -NR⁶CO-B-*, or -CO- other than the carbonyl groups in -CO₂-B-, -OCO-B-, -CONR⁶-B-, and -NR⁶CO-B-, where B is a single bond or C₁₋₁₀-alkylene optionally containing a substituent, R⁶ is H or C₁₋₄-alkyl group optionally containing a substituent. The alkylene group more preferably contains 1-5 carbon atoms. R⁶ is more preferably H or methyl. The symbol * indicates the bond to A in the formula.

L is preferably a single bond.

Examples of the compound include

CH₃C(O)CH₂CH₂CH₂CH₂CH₂CH₂CH₂CH₂COOK,

CH₃C(O)CH₂CH₂CH₂CH₂CH₂CH₂CH₂COONa,

CH₃C(O)CH₂CH₂CH₂CH₂CH₂CH₂COONa,

CH₃C(O)CH₂CH₂CH₂CH₂CH₂COONa,

CH₃C(O)CH₂CH₂CH₂CH₂COONa,

CH₃C(O)CH₂CH₂CH₂COONa,

CH₃C(O)CH₂CH₂CH₂CH₂CH₂CH₂CH₂CH₂CH₂CH₂CH₂CH₂CH₂COONa,

CH₃C(O)CH₂CH₂CH₂CH₂CH₂CH₂CH₂CH₂CH₂CH₂CH₂CH₂CH₂CH₂CH₂CH₂CH₂C H₂COONa,

(CH₃)₃CC(O)CH₂CH₂CH₂CH₂CH₂CH₂CH₂CH₂COONa,

(CH₃)₂CHC(O)CH₂CH₂CH₂CH₂CH₂CH₂CH₂CH₂COONa,

(CH₂)₅CHC(O)CH₂CH₂CH₂CH₂CH₂CH₂CH₂CH₂COONa,

CH₃CH₂C(O)CH₂CH₂CH₂CH₂CH₂CH₂CH₂COONa,

CH₃CH₂CH₂C(O)CH₂CH₂CH₂CH₂CH₂CH₂COONa,

CH₃CH₂CH₂CH₂C(O)CH₂CH₂CH₂CH₂CH₂COONa,

CH₃CH₂CH₂CH₂CH₂C(O)CH₂CH₂CH₂CH₂COONa,

CH₃CH₂CH₂CH₂CH₂CH₂C(O)CH₂CH₂CH₂COONa,

CH₃CH₂CH₂CH₂CH₂CH₂CH₂C(O)CH₂CH₂COONa,

CH₃CH₂CH₂CH₂CH₂CH₂CH₂CH₂C(O)CH₂COONa,

CH₃C(O)CH₂CH₂CH₂CH₂CH₂CH₂CH₂CH₂OCH₂CH₂COONa,

CH₃C(O)CH₂CH₂CH₂CH₂CH₂CH₂CH₂CH₂C(O)NHCH₂COOK,

CH₃C(O)CH₂CH₂CH₂CH₂CH₂CH₂CH₂CH₂NHC(O)CH₂COOK,

CH₃C(O)CH₂CH₂CH₂CH₂CH₂CH₂CH₂CH₂C(O)OCH₂COONa,

CH₃C(O)CH₂CH₂CH₂CH₂CH₂CH₂CH₂CH₂OC(O)CH₂COONa,

CH₃C(O)CH₂CH₂CH₂CH₂CH₂CH₂CH₂CH₂C(O)COONa,

CH₃C(O)CH₂CH₂CH₂CH₂CH₂CH₂CH₂CH₂C(O)COOH,

CH₃C(O)CH₂CH₂CH₂CH₂CH₂CH₂CH₂CH₂C(O)COOLi,

CH₃C(O)CH₂CH₂CH₂CH₂CH₂CH₂CH₂CH₂C(O)COONH₄,

CH₃C(O)CH₂CH₂CH₂CH₂CH₂CH₂CH₂CH₂COONa,

CH₃C(O)CH₂CH₂CH₂CH₂CH₂CH₂CH₂C(CH₃)₂COOK,

CH₃C(O)CH₂CH₂CH₂CH₂CH₂CH₂CH₂CH₂CH₂SO₃Na,

CH₃C(O)CH₂CH₂CH₂CH₂CH₂CH₂CH₂CH₂SO₃Na,

CH₃C(O)CH₂CH₂CH₂CH₂CH₂CH₂CH₂SO₃Na,

CH₃C(O)CH₂CH₂CH₂CH₂CH₂CH₂SO₃Na,

CH₃C(O)CH₂CH₂CH₂CH₂CH₂SO₃Na,

CH₃C(O)CH₂CH₂CH₂CH₂SO₃Na,

CH₃C(O)CH₂CH₂CH₂CH₂CH₂CH₂CH₂CH₂CH₂CH₂CH₂CH₂CH₂SO₃Na,

CH₃C(O)CH₂CH₂CH₂CH₂CH₂CH₂CH₂CH₂CH₂CH₂CH₂CH₂CH₂CH₂CH₂CH₂CH₂C H₂SO₃Na,

CH₃C(O)CH₂CH₂CH₂CH₂CH₂CH₂CH₂CH₂CH₂CH₂SO₃Na,

(CH₃)₃CC(O)CH₂CH₂CH₂CH₂CH₂CH₂CH₂CH₂CH₂SO₃Na,

(CH₃)₂CHC(O)CH₂CH₂CH₂CH₂CH₂CH₂CH₂CH₂CH₂SO₃Na, (CH₂)₅CHC(O) CH₂CH₂CH₂CH₂CH₂CH₂CH₂CH₂CH₂SO₃Na,

CH₃C(O)CH₂CH₂CH₂CH₂CH₂CH₂CH₂CH₂SO₃Na,

CH₃C(O)CH₂CH₂CH₂CH₂CH₂CH₂CH₂SO₃Na,

CH₃C(O)CH₂CH₂CH₂CH₂CH₂CH₂SO₃Na,

CH₃C(O)CH₂CH₂CH₂CH₂CH₂SO₃Na,

CH₃C(O)CH₂CH₂CH₂CH₂SO₃Na,

CH₃C(O)CH₂CH₂CH₂SO₃Na,

CH₃C(O)CH₂CH₂SO₃Na,

CH₃C(O)CH₂SO₃Na,

CH₃C(O)CH₂CH₂CH₂CH₂CH₂CH₂CH₂CH₂CH₂OCH₂CH₂CH₂SO₃Na,

CH₃C(O)CH₂CH₂CH₂CH₂CH₂CH₂CH₂CH₂CH₂C(O)NHCH₂SO₃Na,

CH₃C(O)CH₂CH₂CH₂CH₂CH₂CH₂CH₂CH₂CH₂NHC(O)CH₂SO₃Na,

CH₃C(O)CH₂CH₂CH₂CH₂CH₂CH₂CH₂CH₂CH₂C(O)SO₃Na,

CH₃C(O)CH₂CH₂CH₂CH₂CH₂CH₂CH₂CH₂CH₂C(O)OCH₂SO₃Na,

CH₃C(O)CH₂CH₂CH₂CH₂CH₂CH₂CH₂CH₂CH₂OC(O)CH₂SO₃Na,

CH₃C(O)CH₂CH₂CH₂CH₂CH₂CH₂CH₂CH₂CH₂SO₃H,

CH₃C(O)CH₂CH₂CH₂CH₂CH₂CH₂CH₂CH₂CH₂SO₃K,

CH₃C(O)CH₂CH₂CH₂CH₂CH₂CH₂CH₂CH₂CH₂SO₃Li,

CH₃C(O)CH₂CH₂CH₂CH₂CH₂CH₂CH₂CH₂CH₂SO₃NH₄,

and

CH₃C(O)CH₂CH₂CH₂CH₂CH₂CH₂CH₂CH₂C(CH₃)₂SO₃Na.

The present compound may suitably be produced by a production method including:
a step (11) of reacting a compound (10) of the formula: 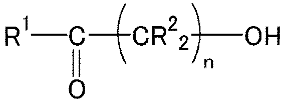 wherein R¹, R², and n are as defined above, and a sultone of the formula: 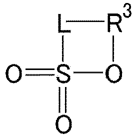 wherein R³ is as defined above; L is as defined above but wherein * indicates the bond to -S(=O)₂- in the formula) to provide a compound (11) of the formula: 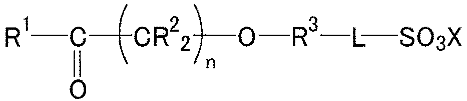 wherein R¹-R³, n, and X are as defined above; L is as defied above, but wherein * indicates the bond to -SO₃X in the formula.

The reaction in step (11) may be performed in the presence of a base.

Examples of the base include sodium hydride, sodium hydroxide, potassium hydroxide, and triethylamine. The base may be used in an amount of 0.5-20 mol relative to 1 mol of the compound (10).

The reaction in step (11) may be performed in a solvent.

The solvent is preferably an organic solvent, more preferably an aprotic polar solvent. Examples of the organic solvent include ethers, aromatic compounds, nitriles, and halogenated hydrocarbons.

Examples of the ethers include diethyl ether, tetrahydrofuran, dioxane, and diethylene glycol diethyl ether. Diethyl ether and tetrahydrofuran are preferred.

Examples of the aromatic hydrocarbons include benzene, toluene, and xylene. Benzene is preferred.

Examples of the nitriles include acetonitrile, propionitrile, butyronitrile, isobutyronitrile, and benzonitrile. Acetonitrile is preferred.

Examples of the halogenated hydrocarbons include dichloromethane, dichloroethane, chloroform, chlorobenzene, and o-dichlorobenzene. Dichloromethane and chloroform are preferred.

The reaction temperature in the step (11) is preferably -78°C to 150°C, more preferably -20°C to 100°C.

The reaction pressure in the step (11) is preferably 0-10 MPa, more preferably 0-1.0 MPa.

The reaction duration in the step (11) is preferably 0.1-72 hours, more preferably 0.1-48 hours.

The present compound may also suitably be produced by a production method including:
a step (21) of oxidizing a compound (20) of the formula: 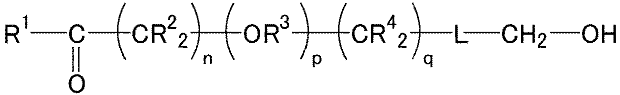 wherein R¹-R⁴, n, p, and q are as defined above; L is as defied above, but wherein * indicates the bond to -CH₂-OH in the formula, to provide a compound (21) of the formula: 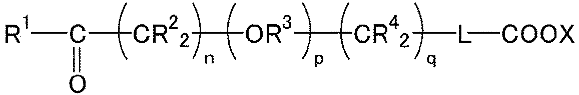 wherein R¹-R⁴, n, p, q, and X are as defined above; and L is as defined above, but wherein * indicates the bond to - COOX in the formula.

The oxidation in step (21) may be performed by allowing a nitrosating agent to act on the compound (20).

Examples of the nitrosating agent include sodium nitrite, nitrosylsulfuric acid, and isoamyl nitrite.

The nitrosating agent may be used in an amount of 0.5-10 mol relative to 1 mol of the compound (20).

The oxidation in step (21) may be performed in a solvent. The solvent used may be trifluoroacetic acid or acetonitrile, for example.

The oxidation temperature in step (21) is preferably -78°C to 200°C, more preferably -20°C to 100°C.

The oxidation pressure in step (21) is preferably 0-10 MPa, more preferably 0-1.0 MPa.

The oxidation duration in step (21) is preferably 0.1-72 hours, more preferably 0.1-24 hours.

The compounds (10) and (20) each may be produced by a production method including:
a step (101) of hydroxylating a compound (100) of the formula:

   R¹¹-CH=CH-Y¹-OH

   wherein R¹¹ is H or linear or branched C₁₋₃-alkyl; and Y¹ is -(CR²₂)ₙ- or -(CR²₂)ₙ-(OR³⁾ₚ-(CR⁴₂)_{q}-L-CH₂-, where R²-R⁴, n, L, p, and q are defined as described above, to provide a compound (101) of the formula: 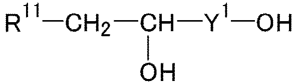
   wherein R¹¹ and Y¹ are as defined above; and
a step (102) of oxidizing the compound (101) to provide a compound (102) of the formula: 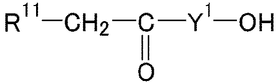 wherein R¹¹ and Y¹ are as defined above.

R¹¹ is preferably H, methyl or ethyl, particularly preferably H or methyl, most preferably H.

The hydroxylation in step (101) may be performed by a method (1) in which iron(II) phthalocyanine (Fe(Pc)) and sodium borohydride are allowed to act on the compound (100) in an oxygen atmosphere or a method (2) in which isopinocampheylborane (IpcBH₂) is allowed to act on the compound (100) and then the resulting intermediate (dialkyl borane) is oxidized.

In method (1), iron(II) phthalocyanine may be used in a catalytic amount, and may be used in an amount of 0.001-1.2 mol relative to 1 mol of the compound (100).

In method (1), sodium borohydride may be used in an amount of 0.5-20 mol relative to 1 mol of the compound (100).

The reaction in method (1) may be performed in a solvent. The solvent is preferably an organic solvent, such as an ether, a halogenated hydrocarbon, an aromatic hydrocarbon, a nitrile, or a nitrogen-containing polar organic compound.

Examples of the ether include diethyl ether, tetrahydrofuran, dioxane, and diethylene glycol diethyl ether. Diethyl ether and tetrahydrofuran are preferred.

Examples of the halogenated hydrocarbon include dichloromethane, dichloroethane, chloroform, chlorobenzene, and o-dichlorobenzene. Dichloromethane and chloroform are preferred.

Examples of the aromatic hydrocarbon include benzene, toluene, and xylene. Benzene and toluene are preferred.

Examples of the nitrile include acetonitrile, propionitrile, butyronitrile, isobutyronitrile, and benzonitrile. Acetonitrile is preferred.

Examples of the nitrogen-containing polar organic compound include N,N-dimethylformamide, N,N-dimethylacetamide, N-methyl-2-pyrrolidone, 2-pyrrolidone, and 1,3-dimethyl-2-imidazolidinone. N,N-dimethylformamide, N,N-dimethylacetamide, and N-methyl-2-pyrrolidone are preferred.

The reaction temperature in method (1) is preferably -78°C to 200°C, more preferably 0-150°C.

The reaction pressure in method (1) is preferably 0-5.0 MPa, more preferably 0.1-1.0 MPa.

The reaction duration in method (1) is preferably 0.1-72 hours, more preferably 0.1-48 hours.

In method (2), isopinocampheylborane may be used in an amount of 1.0-10.0 mol relative to 1 mol of the compound (100).

The reaction of the compound (100) and isopinocampheylborane may be performed in a solvent. The solvent is preferably an organic solvent, such as an ether, a halogenated hydrocarbon, or an aromatic hydrocarbon.

Examples of the ether include diethyl ether, tetrahydrofuran, dioxane, and diethylene glycol diethyl ether. Diethyl ether and tetrahydrofuran are preferred.

Examples of the halogenated hydrocarbon include dichloromethane, dichloroethane, chloroform, chlorobenzene, and o-dichlorobenzene. Dichloromethane and chloroform are preferred.

Examples of the aromatic hydrocarbon include benzene, toluene, and xylene. Benzene and toluene are preferred.

The temperature of the reaction of the compound (100) and isopinocampheylborane is preferably -78°C to 200°C, more preferably 0-150°C.

The pressure of the reaction of the compound (100) and isopinocampheylborane is preferably 0-5.0 MPa, more preferably 0.1-1.0 MPa.

The duration of the reaction of the compound (100) and isopinocampheylborane is preferably 0.1-72 hours, more preferably 0.1-48 hours.

The oxidation in method (2) may be performed by allowing an oxidizing agent to act on the intermediate. An example of the oxidizing agent is hydrogen peroxide. The oxidizing agent may be used in an amount of 0.7-10 mol relative to 1 mol of the intermediate.

The oxidation in method (2) may be performed in a solvent. Examples of the solvent include water, methanol, and ethanol. Water is preferred.

The oxidation temperature in method (2) is preferably 0-100°C, more preferably 0-80°C.

The oxidation pressure in method (2) is preferably 0-5.0 MPa, more preferably 0.1-1.0 MPa.

The oxidation duration in method (2) is preferably 0.1-72 hours, more preferably 0.1-48 hours.

The oxidation of the compound (101) in step (102) may be performed by, for example, (a) a method using the Jones reagent (CrO₃/H₂SO₄) (Jones oxidation), (b) a method using Dess-Martin periodinane (DMP) (Dess-Martin oxidation), (c) a method using pyridinium chlorochromate (PCC), (d) a method of allowing a bleaching agent (about 5-6% aqueous solution of NaOCl) to act in the presence of a nickel compound such as NiCl₂, or (e) a method of allowing a hydrogen acceptor such as an aldehyde or a ketone to act in the presence of an aluminum catalyst such as Al(CH₃)₃ or Al[OCH(CH₃)₂]₃ (Oppenauer oxidation).

The oxidation in step (102) may be performed in a solvent. The solvent is preferably any of water and organic solvents, such as water, ketones, ethers, halogenated hydrocarbons, aromatic hydrocarbons, and nitriles.

Examples of the ketones include acetone, methyl ethyl ketone, methyl isobutyl ketone, cyclohexanone, and diacetone alcohol. Acetone is preferred.

Examples of the ethers include diethyl ether, tetrahydrofuran, dioxane, and diethylene glycol diethyl ether. Diethyl ether and tetrahydrofuran are preferred.

Examples of the halogenated hydrocarbons include dichloromethane, dichloroethane, chloroform, chlorobenzene, and o-dichlorobenzene. Dichloromethane and chloroform are preferred.

Examples of the aromatic hydrocarbons include benzene, toluene, and xylene. Benzene and toluene are preferred.

Examples of the nitriles include acetonitrile, propionitrile, butyronitrile, isobutyronitrile, and benzonitrile. Acetonitrile is preferred.

The oxidation temperature in step (102) is preferably -78°C to 200°C, and may appropriately be selected in accordance with the method used.

The oxidation pressure in step (102) is preferably 0-5.0 MPa, and may appropriately be selected in accordance with the method used.

The oxidation duration in step (102) is preferably 0.1-72 hours, and may appropriately be selected in accordance with the method used.

The compounds (10) and (20) each may also be produced by a production method including:
a step (201) of ozonolyzing a compound (200) of the formula: 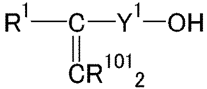 wherein R¹ and Y¹ are as defined above; and R¹⁰¹ is an organic group, to provide a compound (201) of the formula: 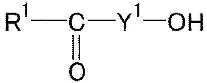 wherein R¹ and Y¹ are as defined above.

R¹⁰¹ preferably each independently is C₁₋₂₀-alkyl.

The ozonolysis in step (201) may be performed by allowing ozone to act on the compound (200), followed by post-treatment with a reducing agent.

The ozone may be generated by dielectric barrier discharge in oxygen gas.

Examples of the reducing agent used in the post-treatment include zinc, dimethyl sulfide, thiourea, and phosphines. Phosphines are preferred.

The ozonolysis in step (201) may be performed in a solvent. The solvent is preferably any of water and organic solvents, such as water, alcohols, carboxylic acids, ethers, halogenated hydrocarbons, and aromatic hydrocarbons.

Examples of the alcohols include methanol, ethanol, 1-propanol, and isopropanol. Methanol and ethanol are preferred.

Examples of the carboxylic acids include acetic acid and propionic acid. Acetic acid is preferred.

Examples of the ethers include diethyl ether, tetrahydrofuran, dioxane, and diethylene glycol diethyl ether. Diethyl ether and tetrahydrofuran are preferred.

Examples of the halogenated hydrocarbons include dichloromethane, dichloroethane, chloroform, chlorobenzene, and o-dichlorobenzene. Dichloromethane and chloroform are preferred.

Examples of the aromatic hydrocarbons include benzene, toluene, and xylene. Benzene and toluene are preferred.

The ozonolysis temperature in step (201) is preferably -78°C to 200°C, more preferably 0-150°C.

The ozonolysis pressure in step (201) is preferably 0-5.0 MPa, more preferably 0.1-1.0 MPa.

The ozonolysis duration in step (201) is preferably 0.1-72 hours, more preferably 0.1-48 hours.

The compounds (10) and (20) each may also be produced by a production method including:
a step (301) of epoxidizing a compound (300) of the formula:

   R²¹-CH=CH-Y¹-OH

   (wherein Y¹ is as defined above; R²¹ is H or linear or branched C₁₋₂-alkyl, to provide a compound (301) of the formula: 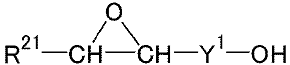
   wherein R²¹ and Y¹ are as defined above;
a step (302) of reacting the compound (301) with a dialkylcopper lithium represented by R²²₂CuLi wherein R²² is C₁₋₂-alkyl, to provide a compound (302) of the formula: 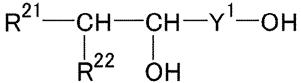 wherein R²¹, R²², and Y¹ are as defined above; and
a step (303) of oxidizing the compound (302) to provide a compound (303) of the formula: 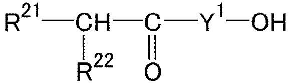 wherein R²¹, R²², and Y¹ are as defined above.

R²¹ is preferably H or a methyl, most preferably H.

R²² is preferably methyl.

R²¹ and R²² preferably contain 1 or 2 carbon atoms, most preferably 1 carbon atom, in total.

The epoxidation in step (301) may be performed by allowing an epoxidizing agent to act on the compound (300).

Examples of the epoxidizing agent include peroxy acids such as meta-chloroperbenzoic acid (m-CPBA), perbenzoic acid, hydrogen peroxide, and tert-butyl hydroperoxide, dimethyl dioxolane, and methyl trifluoromethyl dioxolane. Peroxy acids are preferred, and meta-chloroperbenzoic acid is more preferred.

The epoxidizing agent may be used in an amount of 0.5-10.0 mol relative to 1 mol of the compound (300).

The epoxidation in step (301) may be performed in a solvent. The solvent is preferably an organic solvent, such as a ketone, an ether, a halogenated hydrocarbon, an aromatic hydrocarbon, a nitrile, pyridine, a nitrogen-containing polar organic compound, or dimethyl sulfoxide. Dichloromethane is preferred.

Examples of the ketone include acetone, methyl ethyl ketone, methyl isobutyl ketone, cyclohexanone, and diacetone alcohol. Acetone is preferred.

Examples of the ether include diethyl ether, tetrahydrofuran, dioxane, and diethylene glycol diethyl ether. Diethyl ether and tetrahydrofuran are preferred.

Examples of the halogenated hydrocarbon include dichloromethane, dichloroethane, chloroform, chlorobenzene, and o-dichlorobenzene. Dichloromethane and chloroform are preferred.

Examples of the aromatic hydrocarbon include benzene, toluene, and xylene. Benzene and toluene are preferred.

Examples of the nitrile include acetonitrile, propionitrile, butyronitrile, isobutyronitrile, and benzonitrile. Acetonitrile is preferred.

Examples of the nitrogen-containing polar organic compounds include N,N-dimethylformamide, N,N-dimethylacetamide, N-methyl-2-pyrrolidone, 2-pyrrolidone, and 1,3-dimethyl-2-imidazolidinone. N,N-dimethylformamide, N,N-dimethylacetamide, and N-methyl-2-pyrrolidone are preferred.

The epoxidation temperature in step (301) is preferably -78°C to 200°C, more preferably -40°C to 150°C.

The epoxidation pressure in step (301) is preferably 0-5.0 MPa, more preferably 0.1-1.0 MPa.

The epoxidation duration in step (301) is preferably 0.1-72 hours, more preferably 0.1-48 hours.

In step (302), the dialkylcopper lithium may be used in an amount of 0.5-10.0 mol relative to 1 mol of the compound (301).

The reaction in step (302) may be performed in a solvent. The solvent is preferably an organic solvent, such as an ether, a halogenated hydrocarbon, or an aromatic hydrocarbon.

Examples of the ether include diethyl ether, tetrahydrofuran, dioxane, and diethylene glycol diethyl ether. Diethyl ether and tetrahydrofuran are preferred.

Examples of the halogenated hydrocarbon include dichloromethane, dichloroethane, chloroform, chlorobenzene, and o-dichlorobenzene. Dichloromethane and chloroform are preferred.

Examples of the aromatic hydrocarbon include benzene, toluene, and xylene. Benzene and toluene are preferred.

The reaction temperature in step (302) is preferably -78°C to 200°C, more preferably -40°C to 150°C.

The reaction pressure in step (302) is preferably 0-5.0 MPa, more preferably 0.1-1.0 MPa.

The reaction duration in step (302) is preferably 0.1-72 hours, more preferably 0.1-48 hours.

The oxidation of the compound (302) in step (303) may be performed by, for example, (a) a method of using the Jones reagent (CrO₃/H₂SO₄) (Jones oxidation), (b) a method of using Dess-Martin periodinane (DMP) (Dess-Martin oxidation), (c) a method of using pyridinium chlorochromate (PCC), (d) a method of allowing a bleaching agent (about 5-6% aqueous solution of NaOCl) to act in the presence of a nickel compound such as NiCl₂, or (e) a method of allowing a hydrogen acceptor such as an aldehyde or a ketone to act in the presence of an aluminum catalyst such as Al(CH₃)₃ or Al[OCH(CH₃)₂]₃ (Oppenauer oxidation).

The oxidation in step (303) may be performed in a solvent. The solvent is preferably any of water and organic solvents, such as water, ketones, alcohols, ethers, halogenated hydrocarbons, aromatic hydrocarbons, and nitriles.

Examples of the ketones include acetone, methyl ethyl ketone, methyl isobutyl ketone, cyclohexanone, and diacetone alcohol. Acetone is preferred.

Examples of the alcohols include methanol, ethanol, 1-propanol, and isopropanol. Methanol and ethanol are preferred.

Examples of the ethers include diethyl ether, tetrahydrofuran, dioxane, and diethylene glycol diethyl ether. Diethyl ether and tetrahydrofuran are preferred.

Examples of the halogenated hydrocarbons include dichloromethane, dichloroethane, chloroform, chlorobenzene, and o-dichlorobenzene. Dichloromethane and chloroform are preferred.

Examples of the aromatic hydrocarbons include benzene, toluene, and xylene. Benzene and toluene are preferred.

Examples of the nitriles include acetonitrile, propionitrile, butyronitrile, isobutyronitrile, and benzonitrile. Acetonitrile is preferred.

The oxidation temperature in step (303) is preferably -78°C to 200°C, and may appropriately be selected in accordance with the method used.

The oxidation pressure in step (303) is preferably 0-5.0 MPa, and may appropriately be selected in accordance with the method used.

The oxidation duration in step (303) is preferably 0.1-72 hours, and may appropriately be selected in accordance with the method used.

The compounds (10) and (20) each may also be produced by a production method including:
a step (401) of oxidizing a compound (100) of the formula:

R¹¹-CH=CH-Y¹-OH

wherein R¹¹ and Y¹ are as defined above, to provide a compound (401) of the formula: 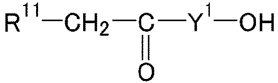 wherein R¹¹ and Y¹ are as defined above.

The oxidation in step (401) may be performed by allowing an oxidizing agent to act on the compound (100) in the presence of water and a palladium compound.

Examples of the oxidizing agent include monovalent or divalent copper salts such as copper chloride, copper acetate, copper cyanide, and copper trifluoromethanethiolate, iron salts such as iron chloride, iron acetate, iron cyanide, iron trifluoromethanethiolate, hexacyanoferrates, and iron sulfate, benzoquinones such as 1,4-benzoquinone, 2,3-dichloro-5,6-dicyano-1,4-benzoquinone, tetrachloro-1,2-benzoquinone, and tetrachloro-1,4-benzoquinone, H₂O₂, MnO₂, KMnO₄, RuO₄, m-chloroperbenzoic acid, peracetic acid, perbenzoic acid, tert-butyl hydroperoxide, cumene hydroperoxide, potassium persulfate, mixtures of potassium hydrogen persulfate-potassium hydrogen sulfate-potassium sulfate, and oxygen. Copper salts, iron salts, benzoquinones, mixtures of potassium hydrogen persulfate-potassium hydrogen sulfate-potassium sulfate, and oxygen are preferred, and copper chloride, iron chloride, 1,4-benzoquinone, and oxygen are more preferred.

The oxidizing agent may be used in an amount of 0.001-10 mol relative to 1 mol of the compound (100).

The water may be used in an amount of 0.5-1000 mol relative to 1 mol of the compound (100).

An example of the palladium compound is palladium dichloride. The palladium compound may be used in a catalytic amount, and may be used in an amount of 0.0001-1.0 mol relative to 1 mol of the compound (100).

The oxidation in step (401) may be performed in a solvent. Examples of the solvent include water, esters, aliphatic hydrocarbons, aromatic hydrocarbons, alcohols, carboxylic acids, ethers, halogenated hydrocarbons, nitrogen-containing polar organic compounds, nitriles, dimethyl sulfoxide, and sulfolane.

Examples of the esters include ethyl acetate, butyl acetate, ethylene glycol monomethyl ether acetate, and propylene glycol monomethyl ether acetate (PGMEA, also known as 1-methoxy-2-acetoxypropane). Ethyl acetate is preferred.

Examples of the aliphatic hydrocarbons include hexane, cyclohexane, heptane, octane, nonane, decane, undecane, dodecane, and mineral spirits. Cyclohexane and heptane are preferred.

Examples of the aromatic hydrocarbons include benzene, toluene, and xylene. Benzene and toluene are preferred.

Examples of the alcohols include methanol, ethanol, 1-propanol, and isopropanol.

Examples of the carboxylic acids include acetic acid and propionic acid. Acetic acid is preferred.

Examples of the ethers include diethyl ether, tetrahydrofuran, dioxane, and diethylene glycol diethyl ether. Diethyl ether and tetrahydrofuran are preferred.

Examples of the halogenated hydrocarbons include dichloromethane, dichloroethane, chloroform, chlorobenzene, and o-dichlorobenzene. Dichloromethane and chloroform are preferred.

Examples of the nitrogen-containing polar organic compounds include N,N-dimethylformamide, N,N-dimethylacetamide, N-methyl-2-pyrrolidone, 2-pyrrolidone, and 1,3-dimethyl-2-imidazolidinone. N,N-dimethylformamide, N,N-dimethylacetamide, and N-methyl-2-pyrrolidone are preferred.

Examples of the nitriles include acetonitrile, propionitrile, butyronitrile, isobutyronitrile, and benzonitrile. Acetonitrile is preferred.

The oxidation temperature in step (401) is preferably -78°C to 200°C, more preferably -20°C to 150°C.

The oxidation pressure in step (401) is preferably 0-10 MPa, more preferably 0.1-5.0 MPa.

The oxidation duration in step (401) is preferably 0.1-72 hours, more preferably 0.1-48 hours.

The present compound may also be produced by a production method including:
a step (31) of oxidizing a compound (30) of the formula:

R¹¹-CH=CH-(CR²₂)ₙ-(OR³)ₚ-(CR⁴₂)_{q}-L-COOX

wherein R²-R⁴, R¹¹, n, p, q, and X are as defined above; and L is as defined above, but wherein * indicates the bond to -COOX in the formula, to provide a compound (31) of the formula: 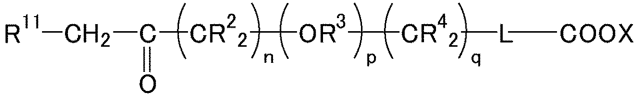
wherein R²-R⁴, L, R¹¹, n, p, q, and X are as defined above.

The oxidation in step (31) may be performed by allowing an oxidizing agent to act on the compound (30) in the presence of water and a palladium compound under the same conditions as in the oxidation in step (401).

The present compound may also be produced by a production method including:
a step (41) of oxidizing a compound (40) of the formula:

   R¹¹-CH=CH- (CR²₂)ₙ-(OR³)ₚ-(CR⁴₂)_{q}-CH=CH₂

   wherein R²-R⁴, R¹¹, n, p, and q are as defined above, to provide a compound (41) of the formula: 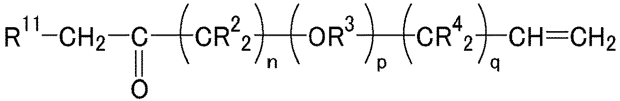
   wherein R²-R⁴, R¹¹, n, p, and q are as defined above; and
a step (42) of reacting the compound (41) with sodium hydrogen subsulfide to provide a compound (42) of the formula: 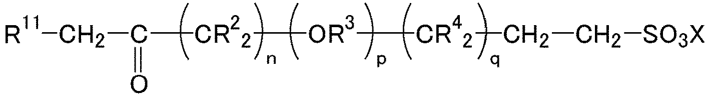 wherein R²-R⁴, R¹¹, n, p, q, and X are as defined above.

The oxidation in step (41) may be performed by allowing an oxidizing agent to act on the compound (40) in the presence of water and a palladium compound under the same conditions as in the oxidation in step (401).

In step (42), the sodium hydrogen subsulfide may be used in an amount of 0.001-20 mol relative to 1 mol of the compound (41).

The reaction in step (42) may be performed in the presence of azobisisobutyronitrile (AIBN).

The azobisisobutyronitrile may be used in an amount of 0.5-20 mol relative to 1 mol of the compound (41).

The reaction in step (42) may be performed in a solvent.

The solvent is preferably an organic solvent, more preferably an aprotic polar solvent. Examples of the organic solvent include ethers, aromatic compounds, nitriles, and halogenated hydrocarbons.

Examples of the ethers include diethyl ether, tetrahydrofuran, dioxane, and diethylene glycol diethyl ether. Diethyl ether and tetrahydrofuran are preferred.

Examples of the aromatic hydrocarbons include benzene, toluene, and xylene. Benzene is preferred.

Examples of the nitriles include acetonitrile, propionitrile, butyronitrile, isobutyronitrile, and benzonitrile. Acetonitrile is preferred.

Examples of the halogenated hydrocarbons include dichloromethane, dichloroethane, chloroform, chlorobenzene, and o-dichlorobenzene. Dichloromethane and chloroform are preferred.

The reaction temperature in step (42) is preferably - 78°C to 150°C, more preferably -20°C to 100°C.

The reaction pressure in step (42) is preferably 0-10 MPa, more preferably 0-1.0 MPa.

The reaction duration in step (42) is preferably 0.1-72 hours, more preferably 0.1-48 hours.

The present compound may also be produced by a production method including:
a step (51) of reacting a compound (50) of the formula:

   R¹¹-CH=CH-(CR²₂)ₙ-(OR³)ₚ-(CR⁴₂)_{q}-Z

   wherein R²-R⁴, R¹¹, n, p, and q are as defined above; and Z is F, Cl, Br or I, with a compound (50-1) of the formula: 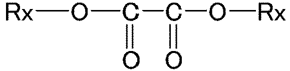
   wherein Rx is linear or branched C₁₋₁₀-alkyl, to provide a compound (51) of the formula: 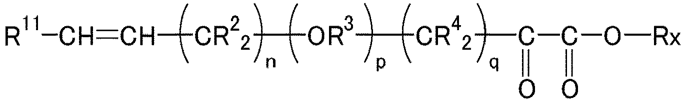
   (wherein R²-R⁴, R¹¹, Rx, n, p, and q are as defined above;
a step (52) of oxidizing the compound (51) to provide a compound (52) of the formula: 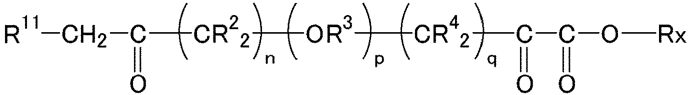 (wherein R²-R⁴, R¹¹, Rx, n, p, and q are as defined above); and
a step (53) of hydrolyzing the compound (52) to provide a compound (53) of the formula: 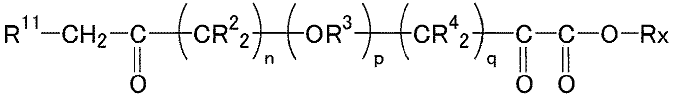 wherein R²-R⁴, R¹¹, Rx, n, p, and q are as defined above.

The reaction in step (51) may be performed in the presence of a metal such as magnesium.

The metal may be used in an amount of 0.5-20 mol relative to 1 mol of the compound (50).

The reaction in step (52) may be performed in a solvent. Examples of the solvent include water, esters, aliphatic hydrocarbons, aromatic hydrocarbons, alcohols, carboxylic acids, ethers, halogenated hydrocarbons, nitrogen-containing polar organic compounds, nitriles, dimethyl sulfoxide, and sulfolane.

Examples of the esters include ethyl acetate, butyl acetate, ethylene glycol monomethyl ether acetate, and propylene glycol monomethyl ether acetate (PGMEA, also known as 1-methoxy-2-acetoxypropane). Ethyl acetate is preferred.

Examples of the aliphatic hydrocarbons include hexane, cyclohexane, heptane, octane, nonane, decane, undecane, dodecane, and mineral spirits. Cyclohexane and heptane are preferred.

Examples of the aromatic hydrocarbons include benzene, toluene, and xylene. Benzene and toluene are preferred.

Examples of the alcohols include methanol, ethanol, 1-propanol, and isopropanol.

Examples of the carboxylic acids include acetic acid and propionic acid. Acetic acid is preferred.

Examples of the ethers include diethyl ether, tetrahydrofuran, dioxane, and diethylene glycol diethyl ether. Diethyl ether and tetrahydrofuran are preferred.

Examples of the halogenated hydrocarbons include dichloromethane, dichloroethane, chloroform, chlorobenzene, and o-dichlorobenzene. Dichloromethane and chloroform are preferred.

Examples of the nitrogen-containing polar organic compounds include N,N-dimethylformamide, N,N-dimethylacetamide, N-methyl-2-pyrrolidone, 2-pyrrolidone, and 1,3-dimethyl-2-imidazolidinone. N,N-dimethylformamide, N,N-dimethylacetamide, and N-methyl-2-pyrrolidone are preferred.

Examples of the nitriles include acetonitrile, propionitrile, butyronitrile, isobutyronitrile, and benzonitrile. Acetonitrile is preferred.

The reaction temperature in step (52) is preferably - 78°C to 150°C, more preferably -20°C to 100°C.

The reaction pressure in step (52) is preferably 0-10 MPa, more preferably 0-1.0 MPa.

The reaction duration in step (52) is preferably 0.1-72 hours, more preferably 0.1-48 hours.

The oxidation in step (52) may be performed by allowing an oxidizing agent to act on the compound (51) in the presence of water and a palladium compound under the same conditions as in the oxidation in step (401).

The hydrolysis in step (53) may be performed by a known method. For example, the hydrolysis may be performed by allowing a sodium hydroxide aqueous solution to act on the compound.

The present compound may also be produced by a production method including:
a step (61) of reacting a compound (60) of the formula: 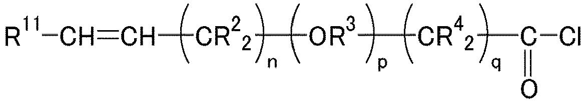 wherein R²-R⁴, R¹¹, n, p, and q are as defined above, with glycine to provide a compound (61) of the formula: 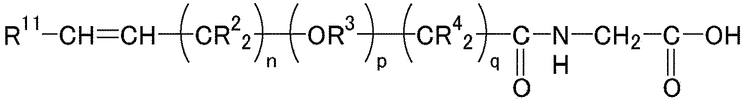 wherein R²-R⁴, R¹¹, n, p, and q are as defined above; and
a step (62) of oxidizing the compound (61) to provide a compound (62) of the formula: 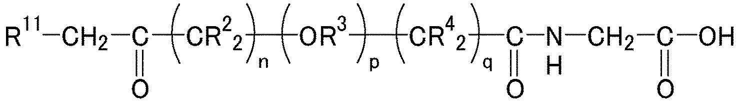 wherein R²-R⁴, R¹¹, n, p, and q are as defined above.

The reaction in step (61) may be performed in the presence of a base.

Examples of the base include sodium hydride, sodium hydroxide, potassium hydroxide, and triethylamine. The base may be used in an amount of 0.5-20 mol relative to 1 mol of the compound (10).

The reaction in step (61) may be performed in a solvent. Examples of the solvent include water, esters, aliphatic hydrocarbons, aromatic hydrocarbons, alcohols, carboxylic acids, ethers, halogenated hydrocarbons, nitrogen-containing polar organic compounds, nitriles, dimethyl sulfoxide, and sulfolane.

Examples of the esters include ethyl acetate, butyl acetate, ethylene glycol monomethyl ether acetate, and propylene glycol monomethyl ether acetate (PGMEA, also known as 1-methoxy-2-acetoxypropane). Ethyl acetate is preferred.

Examples of the aliphatic hydrocarbons include hexane, cyclohexane, heptane, octane, nonane, decane, undecane, dodecane, and mineral spirits. Cyclohexane and heptane are preferred.

Examples of the aromatic hydrocarbons include benzene, toluene, and xylene. Benzene and toluene are preferred.

Examples of the alcohols include methanol, ethanol, 1-propanol, and isopropanol.

Examples of the carboxylic acids include acetic acid and propionic acid. Acetic acid is preferred.

Examples of the ethers include diethyl ether, tetrahydrofuran, dioxane, and diethylene glycol diethyl ether. Diethyl ether and tetrahydrofuran are preferred.

Examples of the halogenated hydrocarbons include dichloromethane, dichloroethane, chloroform, chlorobenzene, and o-dichlorobenzene. Dichloromethane and chloroform are preferred.

Examples of the nitrogen-containing polar organic compounds include N,N-dimethylformamide, N,N-dimethylacetamide, N-methyl-2-pyrrolidone, 2-pyrrolidone, and 1,3-dimethyl-2-imidazolidinone. N,N-dimethylformamide, N,N-dimethylacetamide, and N-methyl-2-pyrrolidone are preferred.

Examples of the nitriles include acetonitrile, propionitrile, butyronitrile, isobutyronitrile, and benzonitrile. Acetonitrile is preferred.

The reaction temperature in step (61) is preferably - 78°C to 150°C, more preferably -20°C to 100°C.

The reaction pressure in step (61) is preferably 0-10 MPa, more preferably 0-1.0 MPa.

The reaction duration in step (61) is preferably 0.1-72 hours, more preferably 0.1-48 hours.

The oxidation in step (62) may be performed by allowing an oxidizing agent to act on the compound (61) in the presence of water and a palladium compound under the same conditions as in the oxidation in step (401).

In each of the above production methods, the resulting compounds may be subjected to any of evaporation of a solvent or operations such as distillation and purification after the respective steps, whereby the purity of each compound may be increased. When the resulting compound is a compound in which X is H, i.e., containing - SO₃H or -COOH, the compound may be brought into contact with an alkali such as sodium carbonate or ammonia so that such a group is converted into a salt form.

### EXAMPLES

The invention is described with reference to examples.

### <Example 1>

A flask was charged with 11-hydroxyundecan-2-one (1.86 g) and THF, and the components were stirred at 0°C. Thereto was added gradually 60% NaH (0.4 g), and the components were then stirred for 30 minutes. A solution of propanesultone (1.22 g) in THF was gradually dropwise added thereto, and the components were then stirred at 40°C for 10 hours. Diethyl ether was added thereto and the mixture was filtered, whereby 1.65 g (yield: 50%) of the target sodium 3-((10-oxoundecyl)oxy)propane-1-sulfonate was obtained.

### <Example 2>

A mixture of 10-undecenoic acid (4.7 g), 1,4-benzoquinone (0.63 g), DMF (50 mL), water (5 mL), and PdCl₂ (0.09 g) was heated and stirred at 90°C for 12 hours.

The solvent was then evaporated under reduced pressure. The resulting residue was mixed with a solution of sodium methoxide in methanol and the mixture was filtered. The solid residue was mixed with hydrochloric acid and the mixture was extracted with ethyl acetate. The extract was dried over sodium sulfate, and the solvent was evaporated under reduced pressure. The residue was purified by column chromatography, whereby 10-oxoundecanoic acid (3.2 g) was obtained.

The spectrum data of the resulting 10-oxoundecanoic acid are the following.
¹H-NMR (CDCl₃) δppm: 1.27-1.37 (m, 8H), 1.51-1.60 (m, 4H), 2.11 (s, 3H), 2.29-2.42 (m, 4H)

The resulting 10-oxoundecanoic acid (0.73 g) was added to a 2 M solution of ammonia in methanol and water was evaporated. Thereby, ammonium 10-oxoundecanoate (0.57 g) was obtained.

The spectrum data of the resulting ammonium 10-oxoundecanoate are the following.
¹H-NMR (CDCl₃) δppm: 1.05 (m, 8H), 1.31 (m, 4H), 1.96-2.02 (m, 5H), 2.28-2.34 (t, J = 7.3, 4H)

### <Example 3>

To 10-oxoundecanoic acid (1.8 g) was added 1.0 M KOH water and water was then evaporated, whereby potassium 10-oxoundecanoate (2.2 g) was obtained.

The spectrum data of the resulting potassium 10-oxoundecanoate are the following.
¹H-NMR (CDCl₃) δppm: 1.04 (m, 8H), 1.30-1.32 (m, 4H), 1.89-2.01 (m, 5H), 2.27-2.33 (t, J = 7.6, 4H)

### <Example 4>

A reactor was charged with 1,10-undecadiene (6.55 g), dimethyl formamide (300 ml), water (43 ml), palladium chloride (0.38 g), and iron(III) sulfate n-hydrate (25.8 g), and the components were heated and stirred at 45°C. The mixture was subjected to liquid separation, dehydration, concentration, and silica gel column chromatography purification, whereby 10-undecen-2-one (3.17 g) was obtained at a yield of 44%.

Next, a reactor was charged with 10-undecen-2-one (1.29 g), methanol (54 ml), water (27 ml), sodium hydrogen sulfite (1.07 g), and azobisisobutyronitrile (0.13 g), and the components were heated and stirred at 80°C. The resulting reaction mixture was recrystallized, whereby sodium 10-oxoundecanesulfonate (0.41 g) was obtained at a yield of 19%.

### <Example 5>

Sodium 11-oxododecanesulfonate was synthesized in the same manner as in Example 4, except that the material was changed from 1,10-undecadiene to 1,11-dodecadiene.

### <Example 6>

A reactor was charged with magnesium (flakes, 8.32 g, 342.1 mmol) and THF (20 mL), and 11-bromoundecene (11.95 g, 102.6 mmol) diluted with THF (100 mL) was slowly dropwise added thereto. The components were then heated up to 70°C. The reaction solution was dropwise added to a solution mixture of diethyl oxalate (10.0 g, 68.4 mmol) and THF (70 mL) cooled at -78°C. The mixture was warmed up to room temperature, and a saturated ammonium chloride aqueous solution (150 mL) was added for quenching. The mixture was subjected to liquid separation, dehydration, concentration, and silica gel column chromatography purification, whereby 8.07 g (yield: 46.3%) of ethyl 2-oxotridec-12-enoate (colorless, transparent liquid) was obtained.

A reactor was charged with ethyl 2-oxotridec-12-enoate (8.62 g, 33.9 mmol), DMAc (20 mL), deionized water (2.0 mL), benzoquinone (3.66 g, 33.9 mmol), and PdCl₂ (30.0 mg, 0.169 mmol), and the components were heated and stirred at 40°C. The mixture was subjected to liquid separation, dehydration, concentration, and silica gel column chromatography purification, whereby 4.54 g (4.54 g) of ethyl 2,12-dioxotridecanoate was obtained at a yield of 50%.

A reactor was charged with ethyl 2,12-dioxotridecanoate (3.63 g, 13.4 mmol) and 1.0 M HCl aqueous solution (50 mL), and the components were heated and stirred at 110°C. The mixture was subjected to liquid separation, dehydration, and concentration, whereby 2,12-dioxotridecanoic acid (3.17 g) was obtained. A 1.0 M NaOH aqueous solution was allowed to act thereon, whereby sodium 2,12-dioxotridecanoate was obtained at a quantitative yield.

### <Example 7>

A reactor was charged with glycine (3.2 g) and sodium hydroxide aqueous solution (85 mL). A solution of 10-undecenoyl chloride (5.7 g) in THF was dropwise added under stirring at 0°C, and the mixture was further stirred at room temperature. The reaction completion was followed by quenching with hydrochloric acid. The product was purified by liquid separation and recrystallization, whereby 2-(10-undecenamide)acetic acid (4.8 g) was obtained at a yield of 71%.

A mixture of 2-(10-undecenamide)acetic acid (4.8 g), N,N-dimethylacetamide (80 mL), water (8.0 mL), and PdCl₂ (0.18 g) was heated and stirred in a reactor in an oxygen atmosphere (0.1 MPa) at 80°C for 24 hours. After the reaction was completed, the product was purified by liquid separation and recrystallization, whereby 2-(10-oxoundecanamide)acetic acid (1.5 g) was obtained at a yield of 29%.

To a solution of this 2-(10-oxoundecanamide)acetic acid (1.5 g) in a mixture of ethanol/water was dropwise added a 0.5 M solution (11 mL) of KOH in ethanol. The mixture was stirred, and the solvent was then evaporated at room temperature under reduced pressure. Thereby, potassium 2-(10-oxoundecanamide)acetate (1.8 g) was obtained in the form of white solid.

The compounds obtained in Examples 1-7 were each dissolved in water so as to have the concentration shown in Table 1, and the surface tension was measured. The surface tension was measured by the Wilhelmy method at 20°C. The results are shown in Table 1.

**[Table 1]**

| Amount of compound relative to water (wt%) | | 0.01 | 0.1 | 1.0 |
|---|---|---|---|---|
| Surface tension (mN/m) | Example 1 | 72.1 | 63.0 | 46.8 |
| | Example 2 | 72.0 | 60.0 | 58.8 |
| | Example 3 | 71.2 | 70.1 | 64.0 |
| | Example 4 | 71.5 | 57.3 | 33.7 |
| | Example 5 | 69.0 | 55.0 | 31.0 |
| | Example 6 | 74.5 | 55.6 | Insoluble and unmeasured |
| | Example 7 | 76.6 | 75.8 | 71.1 |

### INDUSTRIAL APPLICABILITY

The present compound can suitably reduce the surface tension of water.

The present compound can suitably be used as surfactant, surfactant accelerator (particularly as a surfactant accelerator in e.g. coating material, lacquer or adhesive), viscosity reducer, dispersant (particularly an aqueous dispersant), or emulsifier.

## Claims

1. Use of a compound of formula (I): 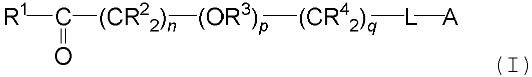 wherein
R¹ is linear or branched C₁₋₄-alkyl;
R², R⁴ each individually are H or linear or branched C₁₋₄-alkyl;
R³ is unsubstituted C₁₋₄-alkylene;
*n* is an integer of ≥ 1;
*p*, *q* each individually are an integer of ≥ 0;
and(*n*+*p*+*q*) is an integer of ≥ 6;
A is -SO₃X or -COOX, wherein X is an alkali metal atom; an alkaline earth metal atom; NR⁵₄ wherein R⁵ each independently is H or an organic group; or imidazolium, pyridinium or phosphonium, each optionally substituted; and
L is a single bond, -CO₂-B-*, -OCO-B-*, -CONR⁶-B-*, -NR⁶CO-B-*, or -CO- other than the carbonyl groups in the aforementioned groups-, B is a single bond or optionally substituted C₁₋₁₀-alkylene, R⁶ is H or optionally substituted C₁₋₄-alkyl, and * indicates the bond to A in the formula;
and the compound contains ≤ 20 carbon atoms in total,
as a surfactant, surfactant accelerator, viscosity reducer, dispersant or emulsifier.

2. The use of claim 1, wherein L in formula (I) is a single bond.

3. The use of claim 1 or 2, wherein R¹ in formula (I) is methyl.

4. The use of any of claims 1-3, wherein X in formula (I) is an alkali metal atom, an alkaline earth metal atom or NR⁵₄.

5. The use of any of claims 1-4, wherein the compound (I) is used as an aqueous dispersant.

## Patentansprüche

1. Verwendung einer Verbindung der Formel (I): 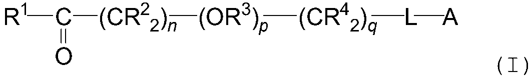 wobei
R¹ ist ein geradkettiges oder verzweigtes C₁₋₄-Alkyl;
R², R⁴ sind jeweils unabhängig voneinander H oder ein geradkettiges oder verzweigtes C₁₋₄-Alkyl;
R³ ist ein unsubstituiertes C₁₋₄-Alkylen;
*n* ist eine ganze Zahl von ≥ 1;
*p, q* sind jeweils unabhängig voneinander eine ganze Zahl von ≥ 0;
und(*n+p+q*) ist eine ganze Zahl von ≥ 6;
A ist -SO₃X oder -COOX, wobei X ist ein Alkalimetallatom; ein Erdalkalimetallatom; NR⁵₄ wobei R⁵ jeweils unabhängig voneinander H oder eine organische Gruppe ist; oder Imidazolium, Pyridinium oder Phosphonium, jeweils gegebenenfalls substituiert; und
L ist eine Einfachbindung, -CO₂-B-*, -OCO-B-*, - CONR⁶-B-*, -NR⁶CO-B-*, oder -CO- mit Ausnahme der Carbonylgruppen in den vorgenannten Gruppen-, B ist eine Einfachbindung oder gegebenenfalls substituiertes C₁₋₁₀-Alkylen, R⁶ ist H oder gegebenenfalls substituiertes C₁₋₄-Alkyl, und * gibt die Bindung an A in der Formel an;
und die Verbindung enthält insgesamt ≤ 20 Kohlenstoffatome,
als Tensid, Tensidbeschleuniger, Viskositätsverringerer, Dispergiermittel oder Emulgator.

2. Verwendung gemäß Anspruch 1, wobei L in Formel (I) eine Einfachbindung ist.

3. Verwendung gemäß Anspruch 1 oder 2, wobei R¹ in Formel (I) Methyl ist.

4. Verwendung gemäß mindestens einem der Ansprüche 1-3, wobei X in Formel (I) ein Alkalimetallatom, ein Erdalkalimetallatom oder NR⁵₄ ist.

5. Verwendung gemäß mindestens einem der Ansprüche 1-4, wobei die Verbindung (I) als wässriges Dispergiermittel verwendet wird.

## Revendications

1. Utilisation d'un composé de formule (I) : 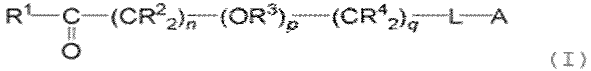 dans laquelle
R¹ est un alkyle en C₁₋₄₋linéaire ou ramifié ;
R², R⁴ sont chacun individuellement H ou un alkyle en C₁₋₄₋linéaire ou ramifié ;
R³ est un alkylène en C₁₋₄- non substitué ;
n est un nombre entier ≥ 1 ;
p, q sont chacun individuellement un nombre entier ≥ 0 ;
et (n+p+q) est un nombre entier ≥ 6 ;
A est -SO₃X ou -COOX, dans laquelle X est un atome de métal alcalin ; un atome de métal alcalino-terreux ; NR⁵₄ dans laquelle R⁵₄ est chacun indépendamment H ou un groupe organique ; ou imidazolium, pyridinium ou phosphonium, chacun éventuellement substitué ; et
L est une liaison simple, -CO₂-B-*, -OCO-B-*, -CONR⁶-B-*, -NR⁶CO-B-*, ou -CO- autre que les groupes carbonyle dans les groupes susmentionnés-, B est une liaison simple ou un alkylène en C₁-₁₀-éventuellement substitué, R⁶ est H ou un alkyle en C₁-₄- éventuellement substitué, et * indique la liaison à A dans la formule ;
et le composé contient au total ≤ 20 atomes de carbone, comme tensioactif, accélérateur de tensioactif, réducteur de viscosité, dispersant ou émulsifiant.

2. Utilisation selon la revendication 1, dans laquelle L dans la formule (I) est une liaison simple.

3. Utilisation selon la revendication 1 ou la revendication 2,
dans laquelle R¹ dans la formule (I) est méthyle.

4. Utilisation selon l'une quelconque des revendications 1 à 3, dans laquelle X dans la formule (I) est un atome de métal alcalin, un atome de métal alcalino-terreux ou NR⁵₄.

5. Utilisation selon l'une quelconque des revendications 1 à 4, dans laquelle le composé (I) est utilisé comme dispersant aqueux.
